# FASCICULE DE BREVET EUROPEEN

(11) **EP 1 149 157 B1**
(45) Date de publication et mention de la délivrance du brevet: **28.05.2008**
(21) Numéro de dépôt: 00901699.9
(22) Date de dépôt: 02.02.2000
(51) Int. Cl.: C12N 1/20, C12Q 1/04, C12Q 1/34

(54) **PROCEDE DE DETECTION DE BACTERIES CULTIVEES EN CONDITION ANAEROBIE**
VERFAHREN ZUM NACHWEIS VON UNTER ANAEROBISCHEN BEDINGUNGEN KULTIVIERTEN BAKTERIEN
METHOD FOR DETECTING BACTERIA CULTIVATED IN ANAEROBIC CONDITION

(30) Priorité: 03.02.1999 FR 9901226
(43) Date de publication de la demande: 31.10.2001
(73) Titulaire: Rambach, Alain, F-75006 Paris (FR)
(72) Inventeur: RAMBACH, Alain, F-75006 Paris (FR); FAVIER, Christine, F-59251 Allennes-les-Marais (FR)
(74) Mandataire: Ahner, Francis
(86) Numéro de dépôt international: PCT/FR2000/000241
(87) Numéro de publication internationale: WO 2000/046345

(56) Documents cités:
- EP-A- 0 238 243
- FR-A- 2 696 476
- FR-A- 2 747 394
- US-A- 3 725 203
- CHEVALIER P ET AL: "X-ALPHA-GAL-BASED MEDIUM FOR SIMULTANEOUS ENUMERATION OF BIFIDOBACTERIA AND LACTIC ACID BACTERIA IN MILK" JOURNAL OF MICROBIOLOGICAL METHODS, vol. 13, no. 1, 1 avril 1991 (1991-04-01), pages 75-83, XP002047086 cité dans la demande
- PATENT ABSTRACTS OF JAPAN vol. 016, no. 238 (C-0946), 2 juin 1992 (1992-06-02) & JP 04 051900 A (NITSUSUI SEIYAKU KK), 20 février 1992 (1992-02-20)

## Description

La présente invention concerne un milieu de culture de bactéries, à utiliser en condition anaérobie, comprenant au moins un complexe métallique qui permet la polymérisation oxydative d'un dérivé indoxyl et un substrat contenant un dérivé indoxyl conduisant à un composé coloré insoluble. Ledit complexe métallique, en particulier le citrate de fer ammoniacal, a une concentration comprise entre 0,3 et 0,9 mg/ml, de préférence 0,6 mg/ml. Avantageusement, le milieu de culture selon l'invention peut comprendre un substrat tel que X-Gal, à une concentration comprise entre 10et500mg/l.

Des nombreuses méthodes d'identification et de dénombrement de souches bactériennes ont été développées pour satisfaire les besoins en tests ou en outils de diagnostic dans tous les domaines techniques, et scientifiques qui touchent à la microbiologie, notamment la médecine et l'agro-alimentaire.

De telles méthodes peuvent s'avérer extrêmement utiles pour le diagnostic des infections opportunistes dont les symptômes sont parfois peu caractéristiques de la cause exacte de la maladie. Par exemple, la maladie de Crohn (MC) est une maladie inflammatoire chronique du tube digestif. Elle se manifeste par des douleurs abdominales, de la diarrhée, de la fièvre et une dénutrition. Les lésions sont caractérisées par une atteinte de la paroi digestive qui est enflammée, épaissie et ulcérée. Cette maladie dure toute la vie, durant laquelle les patients subissent des poussées évolutives suivies de périodes de rémission.

Les travaux consacrés aux modifications de la flore au cours de la MC ont donné des résultats discordants. Cependant, la plupart d'entre eux s'accordent pour conclure à une augmentation du nombre d'*E*. *coli* et de *Bacteroides* du groupe *fragilis.* Aucune souche potentiellement pathogène n'a pu être individualisée.

L'étude d'une glycoprotéine plasmatique excrétée dans les selles, l'oc-1-protéinase inhibiteur, a mis en évidence une altération du métabolisme bactérien chez les patients atteints de MC. En effet, chez les sujets sains, cette glycoprotéine est déglycosylée tout le long du colon suite à l'action des exoglycosidases d'origine bactérienne. Par contre, chez les patients, elle reste glycosylée, ce qui traduit un défaut d'activité de ces osidases. Ce défaut a été effectivement prouvé par des dosages d'activités glycosidasiques au sein d'extraits fécaux. Les activités enzymatiques, et notamment celle de la β-galactosidase, ont été trouvées fortement diminuées chez les patients par rapport aux témoins, Favier et al., (1996), Differenciation and identification of human fecal anaerobic bacteria producing β-galactosidase (a new methodology), Journal of Microgiological Methods 27, 25-31 ; Favier et al., (1997), Fecal β-D-galactosidase Production and Bifidobacteria Are Decreased in Crohn's Disease, Digestive Diseases and Sciences, 42, 817-822.

La capacité de la flore fécale de ces patients et de sujets sains, incubée dans des conditions appropriées, à produire et à libérer la 0-galactosidase, a été étudiée. Pour ce faire, des échantillons fécaux sont cultivés, en atmosphère anaérobie et à 37°C, dans un bouillon Wilkins Chalgren (WC) additionné de mucines gastriques de porc (afin de favoriser la croissance des germes et la production d'exoglycosidases). L'activité β-galactosidase est dosée sur les surnageants d'aliquotes prélevés en début (2h) et fin (22h) d'incubation.

Ainsi, une méthodologie permettant de dénombrer sélectivement les germes anaérobies libérant la β-galactosidase dans les fèces a été développée. Cependant, compte tenu de la complexité de la flore, les méthodes, traditionnellement utilisées, qui consistent à isoler les colonies, identifier les germes, puis doser l'activité enzymatique qu'ils produisent , ne semblent pas susceptibles de répondre au problème posé.

Récemment, l'utilisation de substrats chromogènes, tels que le 5-Bromo-4-Chloro-3-Indolyl-β-D-galactopyranoside ou X-gal, a permis la détection des germes anaérobies Lac⁺. Suite à l'hydrolyse enzymatique, le substrat subit une polymérisation oxydative qui provoque la formation d'un précipité bleu. Cette méthodologie, qui permet de différencier, directement sur boîte de Pétri, les germes capables de libérer la β-gal dans le milieu, a été appliquée à l'étude des germes fécaux de patients et de sujets sains. Les résultats obtenus ont été rapprochés de ceux tirés de l'analyse, à l'aide de milieux sélectifs, des principaux groupes de germes connus pour leur capacité à produire la (3-*galactosidase : Bacteroides, Lactobacillus* et *Bifidobacterium.*
Des exemples de tels milieux sont présentés dans Chevalier, P., Roy, D. and Savoie, L., (1991) X-Gal based medium for simultaneous enumeration of bifidobacteria and lactic acid bacteria in milk, J. Microbiol, Methods 13, 75-83 ; et dans Livingston SJ, Kominos SD, Lee RB, (1978), New medium for selection and presumptive identification of the Bacteroides fragilis group, J. Clin, Microbiol 7, 448-453. Le document US 3.725.203 décrit un milieu de culture permettant l'identification d'entérobactéries de type proteus utilisant une combinaison de L-tryptophane, de citrate de fer ammoniacal et du "Brom cresol purple".

Toutefois, il est parfois difficile de visualiser les bactéries. On a mis au point, dans le cadre de la présente invention, un milieu contenant un complexe métallique oxydant qui permet notamment d'intensifier les halos de couleurs obtenus autour des colonies. Ce perfectionnement de la technique évoquée ci-dessus, a été effectué de façon à favoriser la réaction oxydative du substrat dans le milieu réduit nécessaire à la croissance des bactéries en condition anaérobie.

Ainsi, aucun document de l'art antérieur ne décrit, ni ne suggère la présente invention telle que définie ci-après.

### DESCRIPTION

La présente invention concerne un milieu de culture de bactéries, sélectionné parmi le citrate de fer ammoniacal et un ferricyanide à utiliser en condition anaérobie, comprenant au moins un complexe métallique qui permet la polymérisation oxydative d'un dérivé indoxyl et un substrat contenant un dérivé indoxyl sélectionné parmi ceux cités ci-après conduisant à un composé coloré insoluble. Ledit complexe métallique, en particulier le citrate de fer ammoniacal, a une concentration comprise entre 0,3 et 0,9 mg/ml, de préférence 0,6 mg/ml. Le milieu de culture selon l'invention peut comprendre au moins un sélectionné parmi X-Gal, X-Phos, X-acglmn, Mag-Gal, Mag-α-Gal, et Mag-Phos, de préférence X-Gal, à une concentration comprise entre 10 et 500 mg/l, particulièrement entre 50 et 200 mg/l, de préférence à 100 mg/ml.

On entend par « bactérie » dans le cadre de l'invention, les bactéries anaérobies, aérobie anaérobies, et de toute bactérie produisant naturellement ou non une p-galatosidase. Parmi les bactéries transformées, on peut citer notamment une bactérie transformée par un plasmide contenant le gène LacZ, éventuellement sous le contrôle d'un promoteur d'intérêt.

Dès lors, le milieu selon l'invention est destiné à la détection des bactéries anaérobies, aérobie anaérobies, et de toute bactérie produisant une β-galactosidase.
On peut citer comme exemple les bactéries du genre *Bifidobacterium, Clostridium, Citrobacter, Escherichia,* et/ou *Bacteroides,* en particulier des souches *Bifidobacterium bifidum, Clostridium perfringens, Clostridium butyricum, E. coli,* et/ou *Bacteroides fragilis.*

De préférence, ce milieu de culture comprend le milieu Columbia cystéiné bien connu de l'homme du métier, dont les ingrédients et les caractéristiques sont les suivants (en base qsp selon le fabricant) :

| | |
|---|---|
| Glucose | 5 g |
| Chlorhydrate de cystéine | 0,3 g |
| Agar | 5 g |
| Eau | 1000 ml |
| pH | 7,3 |
| Autoclavage | 15 mm, 120°C |

Néanmoins, le milieu selon l'invention ne se limite pas à une liste d'ingrédients particuliers, de sorte qu'il peut être adapté à la culture d'une bactérie donnée que l'on cherche à détecter. Par exemple, le milieu TSC, décrit ci-dessous, peut servir de base pour la préparation du milieu selon l'invention.

### Milieu TSC (base qsp selon le fabricant):

| | |
|---|---|
| Tryptose | 15 g |
| Peptone de farine de soja | 5 g |
| Extrait de levure | 5 g |
| Disulfite de sodium | 1 g |
| Agar-agar | 15 g |
| Eau | 1000 ml |

Le milieu de culture selon l'invention peut contenir en outre du sulfate de magnésium à une concentration comprise entre 5 mM et 100 mM, de préférence 20 mM, et/ou au moins un antibiotique, par exemple la cyclosérine de préférence à 0,4 g/l, la néomycine additionnée de polymyxine de préférence à 0.02 g/l et 0,05 g/l respectivement.

Un aspect complémentaire de la présente invention concerne un produit de combinaison comprenant au moins un complexe métallique oxydant et au moins un substrat contenant un dérivé indoxyl conduisant à un composé coloré insoluble pour une utilisation simultanée, séparée, ou étalée dans le temps, destiné à la détection de bactéries. Ledit substrat peut-être sélectionné parmi X-Gal, X-Phos, X-acglmn, Mag-Gal, Mag-α-Gal, et Mag-Phos, de préférence X-Gal, et ledit complexe métallique est le citrate de fer ammoniacal.
Ce produit de combinaison est caractérisé en ce que le complexe métallique et le substrat sont véhiculés dans un solvant aqueux à une concentration comprises entre 3 et 900 mg/ml, de préférence à 60 mg/ml, ou un solvant organique à une concentration comprises entre 100mg/l et 50 g/1, particulièrement entre 500 mg/l et 20 g/l, de préférence à 10 g/1. Le produit de combinaison selon l'invention peut contenir en outre du sulfate de magnésium à une concentration comprise entre 50 mM et 10 M, de préférence 2 M, et/ou au moins un antibiotique.
Un aspect avantageux de la présente invention a pour objet une trousse de détection de bactéries comprenant un produit de combinaison tel que défini ci-dessus.

Dans le cadre de l'invention, on entend par « détection » la visualisation, éventuellement l'identification, et la quantification des bactéries.

La présente invention porte également sur un procédé de détection de bactéries caractérisé en ce qu'il comporte les étapes suivantes :
a) On ajoute à un milieu susceptible de contenir lesdites bactéries, cultivées en condition anaérobie, au moins un substrat contenant un dérivé indoxyl conduisant à un composé coloré insoluble,
b) on ajoute au moins un complexe métallique oxydant, en particulier le citrate de fer ammoniacal,
c) on visualise l'apparition d'un précipité de couleur autour des colonies (halo) et/ou une coloration des colonies.

Dans un autre mode de réalisation le procédé de détection de bactéries comporte les étapes suivantes :
a) On cultive lesdites bactéries dans un milieu selon l'invention en condition anaérobie,
b) on visualise l'apparition d'un précipité de couleur autour des colonies (halo) et/ou une coloration des colonies ;
ou encore les étapes suivantes :
a) On ajoute à un milieu, susceptible de contenir lesdites bactéries cultivées en condition anaérobie, un produit de combinaison selon l'invention,
b) on visualise l'apparition d'un précipité de couleur autour des colonies (halo) et/ou une coloration des colonies.

Ledit complexe métallique permet d'intensifier le halo de couleur et/ou pour augmenter la coloration des colonies. En effet, il réagit avec le dérivé indoxyl selon l'invention pour donner un composé coloré qui précipite. L'invention porte également sur l'utilisation d'un milieu, d'un produit de combinaison ou d'un kit tels que décrits ci-dessus pour la détection des bactéries qui possèdent un enzyme permettant la libération d'un dérivé indoxyl à partir d'un substrat contenant un dérivé indoxyl.

L'ajout du citrate de fer ammoniacal permet non seulement de visualiser des couleurs qui n'apparaissent pas en culture dans des jarres en condition anaérobie, mais également d'intensifier le halo de couleurs des colonies cultivées sous sachet plastique en condition anaérobie. Le milieu columbia semble tout à fait avantageux pour l'apparition des couleurs. Bien entendu, les couleurs et l'intensité des couleurs obtenues dépendent étroitement des souches, du niveau d'expression et de sécrétion de la β-galactosidase.

Les exemples ci-après sont donnés afin d'illustrer la présente invention mais ils n'en limitent pas les modalités d'exécution.

### EXEMPLE 1 : MILIEU DESTINE A LA DETECTION DES BIF'IDOBACTERIES

La capacité du citrate de fer ammoniacal (FAC) à augmenter la formation de l'indigo en sachet Generbag Anaer® et en jarre a été déterminée.

Une souche de *Bifidobacterium bifidum* a été ensemencée sur le milieu Columbia cystéiné + X-Gal avec ou sans FAC (0.3 g/l), puis on a testé l'effet de l'ajout du FAC avant ou après autoclavage/régénération.

Les colonies dans le sachet Generbag Anaer® sont entourées par un halo plus intense en présence de FAC, et les halos sont devenus visibles en jarre.
Le dénombrement bactérien montre une diminution du nombre de germes quand le FAC est ajouté au milieu après autoclavage/régénération.

Ainsi, le FAC, ajouté avant régénération du flacon, favorise l'apparition du halo. Différentes concentrations de FAC ont ensuite été testées sur trois souches de *Bifidobacterium.* Les résultats sont présentés au tableau I ci-dessous

**Tableau 1**

| FAC (g/l) | 0 | 0.3 | 0.6 | 0.9 |
|---|---|---|---|---|
| *B. bifidum* | ∅ | + | ++ | +++ ↓ |
| *B. longum* | ∅ | + | ++ | ++ |
| *B. dentium* | ∅ | + | ++ | ++ |

| | | | | |
|---|---|---|---|---|
| ∅ : sans halo + ->+++ : intensité du halo ↓ : diminution du nombre de germe (ici de 90 %) | | | | |

En conséquence, 0.6 g/l de FAC semble être une concentration idéale pour visualiser la présence de halo autour des colonies *Bifidobacterium* après culture dans une jarre.

### EXEMPLE 2 . ETUDE DE DIFFERENTS SUBSTRATS POUR FAIRE APPARAITRE LA COLORATION EN ANAEROBIOSE PAR AJOUT DU FAC.

On a testé différents substrats (100 mg/I) en milieu Columbia cystéiné, en présence et en absence de FAC (0.6 g/I)

### 2.1 Milieu additionné de X-Gal

La présence de FAC a permis d'observer la coloration due à l'hydrolyse du substrat X-Gal pour les colonies de *Clostridium :*
Les colonies de *C*. *perfringens* (β-galactosidase+) sont ocres sans FAC ; bleu-vert avec un halo bleu en présence de FAC. Les colonies de *C*. *butyricum* (β-galactosidase+) sont crèmes sans FAC ; crème-verdâtre entourées d'un léger halo bleu vert avec le FAC. Par contre, les colonies de *Citrobacter* (β-galactosidase+) restent crèmes avec ou sans FAC : soit le FAC ne suffit pas pour voir la coloration, soit les germes n'ont pas hydrolysé le substrat. Dans cette souche le gène LacZ est sans doute sous le contrôle de l'opéron lactose. Dès lors, il est nécessaire d'ajouter du lactose dans le milieu de culture pour induire l'expression de la β-galactosidase.
Les différences entre le milieu avec FAC et le milieu sans FAC sont particulièrement importantes pour les souches de *C*. *perfringens,* et *C*. *butyricum.*

### 2.2 Milieu additionné de Mag-Gal

Les colonies de *C*. *perfringens* (β-galactosidase+) sont ocres sans FAC ; roses avec un halo rose en présence de FAC.
Les colonies de *C*. *butyricum* (β-galactosidase+) sont crèmes sans FAC ; rosées avec un halo rose avec le FAC, et celles de Citrobacter (β-galactosidase+) sont crèmes sans FAC ; rosées avec le FAC. La présence de FAC a permis d'observer la coloration due à l'hydrolyse du substrat Mag-Gal pour les colonies de *Clostridium butyricum* et pour les *Citrobacter.* Les enzymes de ces derniers ne seraient donc pas inductibles mais seraient plus capables d'hydrolyser le Mag-Gal que le X-Gal.
Les différences entre le milieu avec FAC et le milieu sans FAC sont, dans l'ordre, très marquées pour la souche de : *C. perfringens, C. butyricum, Citrobacter.*

### 2.3 Milieu additionné de X-Phos

Les colonies de *C*. *perfringens* (phosphatase alcaline+) sont crèmes sans FAC ; crème verdâtre avec un léger halo bleu en présence de FAC.
Les colonies de *C*. *butyricum* (phosphatase alcaline-) sont crèmes sans FAC ; crèmes avec un halo très clair près de la colonie puis bleu en périphérie avec le FAC, et celles de *Citrobacter* sont crèmes sans FAC ; bleu-vert avec un léger halo bleu-vert avec le FAC.
Les colonies de *E*. *coli* sont crèmes verdâtre très clair sans FAC et plus foncées avec le FAC. Enfin, celles de *Bacteroides fragilis* (phosphatase alcaline+) sont crèmes quand elles sont isolées, bleues en groupe sans FAC ; crème foncé, marron clair avec FAC.

Les différences entre le milieu avec FAC et le milieu sans FAC sont très marquées pour la souche de : *Citrobacter,* viennent ensuite dans l'ordre : *C*. *butyricum, C. perfringens, E. coli* et, enfin *B. fragilis.* Même si les couleurs ne sont pas nettes, la présence de FAC a permis d'observer la coloration (*C*. *perfringens, C. butyricum),* ou d'accroître la couleur *(E. coli)* due à l'hydrolyse du substrat X-Phos. En ce qui concerne *B. fragilis,* cette libère des enzymes extra cellulaires qui forment des halos de couleurs indéfinissables.

### 2.4 Milieu additionné de Mag-Phos

Les colonies de *C*. *perfringens* (phosphatase alcaline+) sont crèmes sans FAC ; roses avec un halo rosé en présence de FAC. Celles de *C*. *butyricum* (phosphatase alcaline-) sont crèmes sans FAC ; roses avec un halo rosé avec le FAC. Celles de *Citrobacter* sont crèmes (centre plus foncé) sans FAC ; roses (gélose rosée) avec le FAC.

Celles de *E*. *coli* sont crèmes (centre plus foncé) sans FAC ; roses (gélose rosée) avec le FAC. Celle de *Bacteroides fragilis* sont rosé-crème sans FAC ; rosé-crème avec un halo brunâtre avec FAC.
La présence de FAC a permis d'observer la coloration due à l'hydrolyse du substrat Mag-Phos pour les colonies de *Clostridium,* les *E*. *coli* et les *Citrobacter.*
Les différences entre le milieu avec FAC et le milieu sans FAC sont plus marquées pour les souches de : *C. perfringens* et *C*. *butyricum,* viennent ensuite *Citrobacter* et *E*. *coli,* enfin *B. fragilis.*

### 2.5 Milieu additionné de Mag-a-Gal

Les colonies de C. perfringens (Mag-α-Gal+) sont crèmes sans FAC ; rosées en présence de FAC. Celles de *Bacteroides fragilis* (Mag-α-Gal+) sont crème foncé sans FAC ; rosé-crème avec FAC. Celles de *C*. *butyricum* (Mag-α-Gal+), *Citrobacter,* et *E*. *coli* sont crèmes sans FAC ; crèmes plus ou moins foncées avec le FAC.
Les différences entre le milieu avec FAC et le milieu sans FAC sont plus marquées pour les souches de : *C. perfringens* et *B. fragilis,* viennent ensuite *C*. *butyricum, Citrobacter* et *E*. *coli.*

### 2.6 Milieu additionné de X-acglmn.

Les colonies de C. *perfringens* (X-acglmn+) sont crèmes sans FAC ; crèmes très légèrement verdâtre en présence de FAC. Celles de *C*. *butyricum* (X-acglmn-) n'ont pas poussé sans FAC ; verdâtres avec un halo bleu en présence de FAC. *Bacteroides fragilis* (X-acglmn+), *Citrobacter* et E. *coli* sont crèmes avec ou sans FAC.
Les différences entre le milieu avec FAC et le milieu sans FAC sont plus marquées pour les souches de : *G perfringens.*

En conclusion, il ressort de ces études que les colorations sont apparues en milieu additionné de FAC, pour la majorité des colonies. Le *C*. *butyricum* donne des résultats contredisant ses activités enzymatiques supposées ; toutefois, la souche utilisée n'est pas forcément représentative de l'espèce.

### EXEMPLE 3 : EXPERIENCES EN MILIEU TSC « NORMAL »

Pour être plus proche des milieux sélectifs permettant les dénombrements des *Clostridii,* on a réalisé une première étude en milieu de base TSC « normal » avec le disulfite et le FAC (1.0 g/l), et sans antibiotique.

Les colonies de *Clostridium perfringens* sont noires. Que l'on ajoute du X-Gal, du Mag-Phos, du X-glu ou du X-glucu, les colorations dues à l'hydrolyse de ces substrats restent difficiles à voir. Cependant, les halos gris-bleu autour des colonies de C. *perfringens* en présence de X-Gal (100 mg/l, associé au Mag-Phos 50 m/l ou au X-glucu 100 m/l) peuvent permettre de distinguer les *C*. *perfringens* des autres germes.
Ces halos sont observés également autour des colonies toutes bleues d'*E*. *coli* (uniquement en X-Gal+ Mag-Phos).

Ainsi, l'apparition de la coloration est gênée par l'utilisation du disulfites par les *C*. *perfringens.* Il semble nécessaire de travailler dans un milieu sans disulfite.

### EXEMPLE 4 : EXPERIENCES EN MILEU TSC SANS DISULFITE

Reprenant la base du milieu TSC (milieu TSC sans antibiotique, et cette fois sans disulfite), on a testé les substrats X-Gal, Mag-Gal, X-Phos et Mag-Phos (100 mg/l) en présence et en absence de FAC (0.6 g/I). Les colorations sont, en général, moins nettes qu'en milieu Columbia.

Avec X-Gal, les différences entre le milieu avec FAC et le milieu sans FAC sont plus marquées pour les souches de : *C*. *perfringens* et *E*. *coli,* puis *Citrobacter* et *B., fragilis. C. butyricum* reste crème avec FAC.
Avec Mag-Gal, les différences entre le milieu avec FAC et le milieu sans FAC sont plus marquées pour la souche de : *C. perfringens* puis pour celles de *E*. *coli* et *Citrobacter,* et enfin *B. fragilis. C. butyricum* n'a pas poussé en absence de FAC.
Avec X-Phos les différences entre le milieu avec FAC et le milieu sans FAC sont plus marquées pour la souche de : *E. coli, Citrobacter* puis celles de *C. perfringens* et *B. fragilis. C. butyricum* n'a pas poussé en absence de FAC.
Avec Mag-Phos les colonies sont juste plus foncées en présence de FAC, les différences entre le milieu avec FAC et le milieu sans FAC sont plus marquées que les souches de *E*. *coli* et *Citrobacter* puis pour celle de *C. perfringens.*

En conclusion, il est préférable d'utiliser le milieu Columbia cystéiné pour faire apparaître une couleur plus intense comparé au milieu TSC.
Si on donne une valeur pour l'intensité des couleurs des colonies en fonction du substrat, la β-galactosidase semble permettre de visualiser d'avantage les C. *perfringens* par rapport à la phosphatase (Voir tableau II ci-dessous)

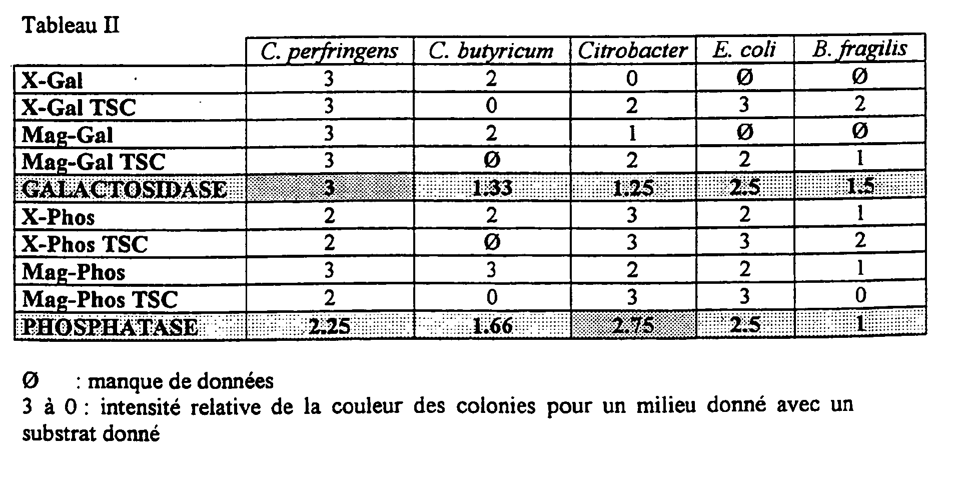

### EXEMPLE 5 : ETUDE DE L'EFFICACITE DE L'AJOUT DES FERRI-CYANIDES POUR FAIRE APPARAITRE LA COLORATION EN ANAEROBIOSE

On a utilisé le ferri cyanide seul à 0.6 g/l. Il n'y a aucune différence entre les milieux contenant ou non ce produits pour le substrat X-Gal.
Seules les colonies de Bacteroides fragilis sont bleues avec le X-Phos en présence de ferri cyanide.

Ainsi, le X-Phos + ferri cyanide peut être un excellent milieu pour pré-identifier les Bacteroides.

### EXEMPLE 6 : AMELIORATION DU MILIEU DESTINE A L'ETUDE DES CLOSTRIDII PAR AJOUT D'ANTIBIOTIQUES

Pour se rapprocher du milieu TSC sélectionnant les C. *perfringens,* on a ajouté'des antibiotiques à la base TSC :
- soit de la cyclosérine (0.4 g/l) ;
- soit de la néomycine additionnée de polymyxine (0.02 et 0.05 g/l respectivement) ;
- soit les trois ensemble.

Toutes les colonies (*C. perfringens, C. butyricum, E. coli, B. fragilis, Citrobacter)* poussent en présence de cyclosérine seule. Par contre, l'association néomycine et polymyxine (0.02 g/l et 0.05 g/l) permet d'inhiber la croissance de *B. fragilis, E. coli* et *Citrobacter.*
Les deux souches suivantes de *Clostridium* poussent :
- les colonies de *C*. *butyricum* restent crèmes,
- celles de *C*. *perfringens* sont légèrement colorée (colonies au centre rosé avec Mag-Gal (100 mg/l) et FAC (0.6 g/l) et colonies au centre verdâtre entourées d'un très léger halo avec X-Gal (100 mg/l) et FAC (0.6 g/l)).

En milieu Columbia cystéiné, les couleurs devraient être plus foncées et l'addition d'antibiotiques, qui possèdent le grand avantage d'être autoclavables, devrait permettre d'inhiber les autres germes.

La croissance des *C*. *butyricum* n'est pas gênante en milieu TSC étant donné que les colonies restent crèmes, mais sera gênante en milieu Columbia car les colonies ont des colorations proches de celles des *C*. *perfringens.*

## Revendications

1. Milieu de culture de bactéries, à utiliser en condition anaérobie, comprenant au moins un complexe métallique sélectionné parmi le citrate de fer ammoniacal et un ferricyanide permettant la polymérisation oxydative d'un dérivé indoxyl et un substrat contenant un dérivé indoxyl conduisant à un composé coloré insoluble sélectionné parmi X-Gal, X-Phos, X-acglmn, Mag-GaI, Mag-a-Gal, et Mag-Phos.

2. Milieu de culture selon la revendication 1 dans lequel ledit complexe métallique a une concentration comprise entre 0,3 et 0,9 mg/ml, de préférence 0,6 mg/ml.

3. Milieu de culture selon l'une des revendications 1 et 2 dans lequel ledit complexe métallique est le citrate de fer ammoniacal.

4. Milieu de culture selon la revendication 1 dans lequel ledit substrat est X-Gal.

5. Milieu de culture selon la revendication 4 dans lequel ledit substrat a une concentration comprise entre 10 et 500 mg/l, particulièrement entre 50 et 200 mg/l, de préférence à 100 mg/ml.

6. Milieu de culture selon l'une des revendications 1 à 5 **caractérisé en ce qu'**il est destiné à la détection des bactéries anaérobies, aérobie anaérobies, et de toute bactérie produisant une β-galactosidase.

7. Milieu de culture selon la revendication 6 **caractérisé en ce qu'**il est destiné à la culture des bactéries du genre *Bifidobacterium, Clostridium, Citrobacter, Escherichia,* et/ou *Bacteroides,* en particulier des souches *Bifidobocterium bifidum, Clostridium perfringens, Clostridium butyricum, E. coli,* et/ou *Bacteroides* fragiles.

8. Milieu de culture selon la revendication 7 **caractérisé en ce qu'**il comprend le milieu Columbia cystéiné.

9. Milieu de culture selon l'une des revendications 1 à 8 **caractérisé en ce qu'**il comporte en outre du sulfate de magnésium à une concentration comprise entre 5 mM et 100 mM, de préférence 20 mM, et/ou au moins un antibiotique.

10. Kit de détection de bactéries définies à la revendication 6 ou 7 comprenant au moins un complexe métallique oxydant sélectionné parmi le citrate de fer ammoniacal et un ferricyanide et au moins un substrat contenant un dérivé indoxyl sélectionné parmi X-Gal, X-Phos, X-acglmn, Mag-Gal, Mag-a-GaI, et Mag-Phos conduisant à un composé coloré insoluble.

11. Kit selon la revendication 10 **caractérisé en ce que** ledit substrat est X-Gal.

12. Kit selon l'une des revendications 10 et 11 **caractérisé en ce que** ledit complexe métallique est le citrate de fer ammoniacal.

13. Kit selon l'une des revendications 10 à 12 **caractérisé en ce que** ledit complexe métallique et ledit substrat sont véhiculés dans un solvant aqueux à une concentration comprises entre 3 et 900 mg/ml, de préférence à 60 mg/ml, ou un solvant organique à une concentration comprises entre 100mg/l et 50 g/l, particulièrement entre 500 mg/l et 20 g/l, de préférence à 10 g/l.

14. Kit selon l'une des revendications 10 à 13 **caractérisé en ce qu'**il comporte en outre du sulfate de magnésium à une concentration comprise entre 50 mM et 10 M, de préférence 2 M, et/ou au moins un antibiotique.

15. Procédé de détection de bactéries **caractérisé en ce qu'**il comporte les étapes suivantes :
a) on ajoute à un milieu susceptible de contenir les bactéries définies à la revendication 6 ou 7, cultivées en condition anaérobie, au moins un substrat contenant un dérivé indoxyl sélectionné parmi X-Gal, X-Phos, X-acglmn, Mag-Gal, Mag-a-Gal, et Mag-Phos conduisant à un composé coloré insoluble,
b) on ajoute au moins un complexe métallique oxydant sélectionné parmi le citrate de fer ammoniacal et un ferricyanide,
c) on visualise l'apparition d'un précipité de couleur autour des colonies (halo) et/ou une coloration des colonies.

16. Procédé de détection de bactéries **caractérisé en ce qu'**il comporte les étapes suivantes : .
a) on cultive les bactéries définies à la revendication 6 ou 7 dans un milieu selon l'une des revendications 1 à 9 en condition anaérobie,
b) on visualise l'apparition d'un précipité de couleur autour des colonies (halo) et/ou une coloration des colonies.

## Claims

1. Bacterial culture medium, for use under anaerobic conditions, comprising at least one metal complex, selected from ammoniacal iron citrate and a ferricyanide, which allows the oxidative polymerization of an indoxyl derivative and a substrate containing an indoxyl derivative, resulting in an insoluble coloured compound which is selected from X-Gal, X-Phos, X-acglmn, Mag-Gal, Mag-a-Gal, and Mal-Phos.

2. Culture medium according to Claim 1, in which said metal complex has a concentration of between 0.3 and 0.9 mg/ml, preferably 0.6 mg/ml.

3. Culture medium according to either of Claims 1 and 2, in which said metal complex is ammoniacal iron citrate.

4. Culture medium according to Claim 1, in which said substrate is X-Gal.

5. Culture medium according to Claim 4, in which said substrate has a concentration of between 10 and 500 mg/l, particularly between 50 and 200 mg/l, preferably at 100 mg/ml.

6. Culture medium according to one of Claims 1 to 5, **characterized in that** it is intended for the detection of anaerobic bacteria, aerobic anaerobic bacteria and any bacterium producing a β-galactosidase.

7. Culture medium according to Claim 6, **characterized in that** it is intended for culturing bacteria of the genus *Bifidobacterium, Clostridium, Citrobacter, Escherichia,* and/or *Bacteroides,* in particular of the strains *Bifidobacterium bifidum, Clostridium perfringens, Clostridium butyricum, E. coli*, and/or *Bacteroides fragilis.*

8. Culture medium according to Claim 7, **characterized in that** it comprises cysteinated Columbia medium.

9. Culture medium according to one of Claims 1 to 8, **characterized in that** it comprises, in addition, magnesium sulphate at a concentration of between 5 mM and 100 mM, preferably 20 mM, and/or at least one antibiotic.

10. Kit for the detection of bacteria defined in Claims 6 or 7, comprising at least one oxidizing metal complex, selected from ammoniacal iron citrate and a ferricyanide, and at least one substrate containing an indoxyl derivative which is selected from X-Gal, X-Phos, X-acglmn, Mag-Gal, Mag-a.-Gal, and Mag-Phos resulting in an insoluble coloured compound.

11. Kit according to Claim 10, **characterized in that** said substrate is X-Gal.

12. Kit according to either of Claims 10 and 11, **characterized in that** said metal complex is ammoniacal iron citrate.

13. Kit according to one of Claims 10 to 12, **characterized in that** said metal complex and said substrate are carried in an aqueous solvent at a concentration of between 3 and 900 mg/ml, preferably at 60 mg/ml, or an organic solvent at a concentration of between 100 mg/l and 50 g/l, particularly between 500 mg/l and 20 g/l, preferably at 10 g/l.

14. Kit according to one of Claims 10 to 13, **characterized in that** it comprises, in addition, magnesium sulphate at a concentration of between 50 mM and 10 M, preferably 2 M, and/or at least one antibiotic.

15. Method for the detection - of bacteria, **characterized in that** it comprises the following steps:
a) there are added to a medium which may contain the bacteria defined in Claim 6 or 7, cultured under anaerobic conditions, at least one substrate containing an indoxyl derivative selected from X-Gal, X-Phos, X-acglmn, Mag-Gal, Mag-a-Gal, and Mag-Phos resulting in an insoluble coloured compound,
b) at least one oxidizing metal complex, selected from ammoniacal iron citrate and a ferricyanide, is added,
c) the appearance of a coloured precipitate around the colonies (halo) and/or a colour of the colonies is visualized.

16. Method for the detection of bacteria, **characterized in that** it comprises the following steps:
a) the bacteria defined in Claim 6 or 7 are cultured in a medium according to one of Claims 1 to 9 under anaerobic conditions,
b) the appearance of a coloured precipitate around the colonies (halo) and/or a colour of the colonies is visualized.

## Patentansprüche

1. Kulturmedium für Bakterien zur Verwendung unter anaeroben Bedingungen, umfassend mindestens einen metallischen Komplex, ausgewählt unter Eisenammoniumcitrat und einem Eisen(III)-cyanid, welches die oxidative Polymerisation eines Indoxyl-Derivats und eines ein Indoxyl-Derivat enthaltenden Substrats, welches zu einer gefärbten unlöslichen Verbindung führt, ausgewählt aus X-Gal, X-Phos, X-acglmn, Mag-Gal, Mag-α-Gal und Mag-Phos, erlaubt.

2. Kulturmedium nach Anspruch 1, in welchem der metallische Komplex eine Konzentration zwischen 0,3 und 0,9 mg/ml eingeschlossen, vorzugsweise 0,6 mg/ml hat.

3. Kulturmedium nach einem der Ansprüche 1 und 2, in welchem der metallische Komplex Eisenammoniumcitrat ist.

4. Kulturmedium nach Anspruch 1, in welchem das Substrat X-Gal ist.

5. Kulturmedium nach Anspruch 4, in welchem das Substrat eine Konzentration zwischen 10 und 500 mg/l eingeschlossen, insbesondere zwischen 50 und 200 mg/l, vorzugsweise von 100 mg/ml hat.

6. Kulturmedium nach einem der Ansprüche 1 bis 5, **dadurch gekennzeichnet, dass** es für den Nachweis von anaeroben Bakterien, aerob-anaeroben Bakterien und eines jeglichen Bakteriums, welches eine β-Galactosidase produziert, bestimmt ist.

7. Kulturmedium nach Anspruch 6, **dadurch gekennzeichnet, dass** es für die Kultur von Bakterien der Gattung *Bifidobacterium, Clostridium, Citrobacter, Escherichia* und/oder *Bacteroides,* insbesondere der Stämme *Bifidobacterium bifidum, Clostridium perfringens, Clostridium butyricum, E. coli* und/oder *Bacteroides fragilis* bestimmt ist.

8. Kulturmedium nach Anspruch 7, **dadurch gekennzeichnet, dass** es das mit Cystein versetzte Columbia-Medium umfasst.

9. Kulturmedium nach einem der Ansprüche 1 bis 8, **dadurch gekennzeichnet, dass** es außerdem Magnesiumsulfat in einer Konzentration zwischen 5 mM und 100 mM eingeschlossen, vorzugsweise 20 mM, und/oder mindestens ein Antibiotikum umfasst.

10. Kit zum Nachweis von Bakterien, die in Anspruch 6 oder 7 definiert sind, umfassend mindestens einen oxidierenden metallischen Komplex, ausgewählt unter Eisenammoniumcitrat und einem Eisen(III)-cyanid, und mindestens ein Substrat, enthaltend ein Indoxyl-Derivat, ausgewählt unter X-Gal, X-Phos, X-acglmn, Mag-Gal, Mag-a,-Gal und Mag-Phos, welches zu einer gefärbten unlöslichen Verbindung führt.

11. Kit nach Anspruch 10, **dadurch gekennzeichnet, dass** das Substrat X-Gal ist.

12. Kit nach einem der Ansprüche 10 und 11, **dadurch gekennzeichnet, dass** der metallische Komplex Eisenammoniumcitrat ist.

13. Kit nach einem der Ansprüche 10 bis 12, **dadurch gekennzeichnet, dass** der metallische Komplex und das Substrat sich in einem wässrigen Lösemittel als Träger in einer Konzentration zwischen 3 und 900 mg/ml eingeschlossen, vorzugsweise von 60 mg/ml oder einem organischen Lösemittel als Träger in einer Konzentration zwischen 100 mg/l und 50 g/l eingeschlossen, insbesondere zwischen 500 mg/l und 20 g/l, vorzugsweise von 10 g/l befinden.

14. Kit nach einem der Ansprüche 10 bis 13, **dadurch gekennzeichnet, dass** er außerdem Magnesiumsulfat in einer Konzentration zwischen 50 mM und 10 M eingeschlossen, vorzugsweise 2 M, und/oder mindestens ein Antibiotikum umfasst.

15. Verfahren zum Nachweis von Bakterien, **dadurch gekennzeichnet, dass** es die folgenden Schritte umfasst:
a) man setzt zu einem Medium, das die in Anspruch 6 oder 7 definierten Bakterien, kultiviert unter anaeroben Bedingungen, enthalten könnte, mindestens ein Substrat, enthaltend ein Indoxyl-Derivat, ausgewählt unter X-Gal, X-Phos, X-acglmn, Mag-Gal, Mag-α-Gal und Mag-Phos, welches zu einer gefärbten unlöslichen Verbindung führt, zu,
b) man setzt mindestens einen oxidierenden metallischen Komplex, welcher unter Eisenammoniumcitrat und einem Eisen(111)-cyanid ausgewählt ist, zu,
c) man visualisiert das Auftreten eines farbigen Präzipitats um die Kolonien herum (Hof) und/oder eine Färbung der Kolonien.

16. Verfahren zum Nachweis von Bakterien, **dadurch gekennzeichnet, dass** es die folgenden Schritte umfasst:
a) man kultiviert die in Anspruch 6 oder 7 definierten Bakterien in einem Medium nach einem der Ansprüche 1 bis 9 unter anaeroben Bedingungen,
b) man visualisiert das Auftreten eines farbigen Präzipitats um die Kolonien herum (Hof) und/oder eine Färbung der Kolonien.
